(19) **Europäisches Patentamt / European Patent Office / Office européen des brevets**

(11) **EP 2 686 435 B1**

(12) **EUROPEAN PATENT SPECIFICATION**

(45) Date of publication and mention of the grant of the patent:
**15.06.2016 Bulletin 2016/24**

(21) Application number: **12710906.4**

(22) Date of filing: **16.03.2012**

(51) Int Cl.:
**C12N 5/077** *(2010.01)*      **A61K 35/32** *(2006.01)*
**C12P 19/04** *(2006.01)*

(86) International application number:
**PCT/EP2012/054654**

(87) International publication number:
**WO 2012/126824 (27.09.2012 Gazette 2012/39)**

(54) **CHONDROGENIC DIFFERENTIATION MEDIA AND METHODS FOR INDUCING CHONDROGENIC DIFFERENTIATION OF CELLS**

CHONDROGENE DIFFERENZIERUNGSMEDIEN UND VERFAHREN ZUR INDUZIERUNG DER CHONDROGENEN DIFFERENZIERUNG VON ZELLEN

MILIEU DE DIFFÉRENCIATION CHONDROGÈNE ET PROCÉDÉ D'INDUCTION DE DIFFÉRENCIATION CHONDROGÈNE DES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2011 EP 11305306**

(43) Date of publication of application:
**22.01.2014 Bulletin 2014/04**

(73) Proprietors:
• **INSERM (Institut National de la Santé et de la Recherche Médicale)**
  **75013 Paris Cédex 13 (FR)**
• **Institut Francais de Recherche pour l'Exploitation de la Mer (IFREMER)**
  **92130 Issy-les-Moulineaux (FR)**
• **Université de Nantes**
  **44035 Nantes (FR)**

(72) Inventors:
• **REDERSTORFF, Emilie**
  **68640 Feldbach (FR)**
• **SINQUIN, Corinne**
  **44300 Nantes (FR)**
• **RATISKOL, Jacqueline**
  **44980 Sainte Luce Sur Loire (FR)**
• **COLLIEC-JOUAULT, Sylvia**
  **44100 Nantes (FR)**
• **GUICHEUX, Jérome**
  **44042 Nantes (FR)**

• **WEISS, Pierre**
  **44800 Saint Herblain (FR)**
• **MERCERON, Christophe**
  **44000 Nantes (FR)**

(74) Representative: **Cabinet Plasseraud**
  **66, rue de la Chaussée d'Antin**
  **75440 Paris Cedex 09 (FR)**

(56) References cited:
**WO-A1-2009/070842**

• **VINATIER C ET AL: "A silanized hydroxypropyl methylcellulose hydrogel for the three-dimensional culture of chondrocytes", BIOMATERIALS, ELSEVIER SCIENCE PUBLISHERS BV., BARKING, GB, vol. 26, no. 33, 1 November 2005 (2005-11-01), pages 6643-6651, XP27767631, ISSN: 0142-9612, DOI: DOI:10.1016/J.BIOMATERIALS.2005.04.057**
• **REDERSTORFF E ET AL: "An in vitro study of two GAG-like marine polysaccharides incorporated into injectable hydrogels for bone and cartilage tissue engineering.", ACTA BIOMATERIALIA MAY 2011 LNKD- PUBMED:21256989, vol. 7, no. 5, 21 January 2011 (2011-01-21), pages 2119-2130, XP002653209, ISSN: 1878-7568**

**(Cont. next page)**

- REDERSTORFF E ET AL: "A self-setting hydrogel doped with an exopolysaccharide from marine origin as a scaffold for cartilage tissue engineering", BONE (NEW YORK), vol. 48, no. Suppl. 2, May 2011 (2011-05), page S141, XP002653210, & 3RD JOINT MEETING OF THE EUROPEAN-CALCIFIED-TISSUE-SOCIETY/INTE RNAT IO NAL-BONE-AND-MINERAL-SOCIETY; ATHENS, GREECE; MAY 07 -11, 2011
- ZANCHETTA P ET AL: "A new bone-healing material: A hyaluronic acid-like bacterial exopolysaccharide", CALCIFIED TISSUE INTERNATIONAL, NEW YORK, NY, US, vol. 72, no. 1, 1 January 2003 (2003-01-01), pages 74-79, XP002402109, ISSN: 0171-967X, DOI: DOI:10.1007/S00223-001-2091-X [retrieved on 2002-10-10]
- GUEZENNEC J ET AL: "Sulfation and depolymerization of a bacterial exopolysaccharide of hydrothermal origin", CARBOHYDRATE POLYMERS, APPLIED SCIENCE PUBLISHERS, LTD. BARKING, GB, vol. 37, no. 1, 1 September 1998 (1998-09-01), pages 19-24, XP004141130, ISSN: 0144-8617, DOI: DOI:10.1016/S0144-8617(98)00006-X
- PUETZER JENNIFER L ET AL: "Comparative Review of Growth Factors for Induction of Three-Dimensional In Vitro Chondrogenesis in Human Mesenchymal Stem Cells Isolated from Bone Marrow and Adipose Tissue", TISSUE ENGINEERING PART B-REVIEWS, vol. 16, no. 4, August 2010 (2010-08), pages 435-444, XP002653211, ISSN: 1937-3368
- C. RUIZ VELASCO ET AL: "Effects of a sulfated exopolysaccharide produced by Altermonas infernus on bone biology", GLYCOBIOLOGY, vol. 21, no. 6, 1 June 2011 (2011-06-01), pages 781-795, XP055003539, ISSN: 0959-6658, DOI: 10.1093/glycob/cwr002

SEQUENCE LISTING

    <110> INSERM (Institut National de la Santé et de la Recherche Médicale) INSTITUT FRANCAIS DE RECHERCHE POUR L'EXPLOITATION DE LA MER (IFREMER)

<120> CHONDROGENIC DIFFERENTIATION MEDIA AND METHODS FOR INDUCING CHONDROGENIC DIFFERENTIATION OF CELLS

<130> BEP110081EP

<160> 10

<170> PatentIn version 3.5

<210> 1
<211> 18
<212> DNA
<213> Artificial Sequence

<220>
<223> Primer

<400> 1
ccaaccgcga gaagatga 18

<210> 2
<211> 20
<212> DNA
<213> Artificial Sequence

<220>
<223> Primer

<400> 2
ccagaggcgt acagggatag 20

<210> 3
<211> 18
<212> DNA
<213> Artificial Sequence

<220>
<223> Primer

<400> 3
tgtcagggcc aggatgtc 18

<210> 4
<211> 22
<212> DNA
<213> Artificial Sequence

<220>
<223> Primer

<400> 4
atcattatac ctctgcccat cc 22

<210> 5
<211> 20
<212> DNA

<213> Artificial Sequence

<220>
<223> Primer

<400> 5
cctccccttc acgtgtaaaa        20

<210> 6
<211> 20
<212> DNA
<213> Artificial Sequence

<220>
<223> Primer

<400> 6
gctccgcttc tgtagtctgc 20

<210> 7
<211> 19
<212> DNA
<213> Artificial Sequence

<220>
<223> Primer

<400> 7
gtacccgcac ttgcacaac 19

<210> 8
<211> 21
<212> DNA
<213> Artificial Sequence

<220>
<223> Primer

<400> 8
tcgctctcgt tcagaagtct c 21

<210> 9
<211> 18
<212> DNA
<213> Artificial Sequence

<220>
<223> Primer

<400> 9
gcaccgacgt caacgagt 18

<210> 10
<211> 20
<212> DNA
<213> Artificial Sequence

<220>

<223> Primer

<400> 10
tggtgttgat acagcggact        20

**Description**

**Background of the Invention**

Articular cartilage is a specialized tissue that surrounds the ends of long bones. It reduces friction and acts as shock-absorbing tissue during joint mobilization. Cartilage is composed of a single cell type, the chondrocyte, that is responsible for the synthesis of an abundant extracellular matrix essentially composed of type II, IX and XI collagens and proteoglycans. Chondrocytes play a major role in maintaining the integrity of cartilage through the control of anabolic and catabolic processes. Cartilage is however susceptible to damage originating from aging, trauma, and inflammatory or degenerative diseases. These impairments often result in extracellular matrix degradation and ultimately in the loss of joint function.

Many surgical approaches have been developed to improve the poor intrinsic self-repair capacity of cartilage. Unfortunately, these techniques have not proved satisfactory effectiveness. In this context, the regeneration of a functional cartilage through the transplantation of mesenchymal stem cells with bioactive synthetic matrices has been contemplated. Mesenchymal stem cells (MSCs) have initially been identified in bone marrow and, since, in various other tissues such as for example adipose tissue, tendon, synovial membrane, muscle, periosteum. MSCs exhibit a number of attractive features, particularly for regenerative medicine, including their self-renewal, their ability to proliferate in culture and their multipotency. In particular, bone-marrow-derived mesenchymal stem cells and adipose-derived stem cells (ASCs) show promise for use in cartilage repair. However, greater understanding is needed to develop a consistently reproducible approach to potently induce chondrogenic differentiation in these stem cell lines and to inhibit differentiated chondrocytes from further maturation into the hypertrophic lineage.

One approach to induce chondrogenic differentiation has been to use hydrogels incorporating polysaccharides, including exopolysaccharides from marine origin (WO 2009/070842; Vinatier et al., Biomaterials, 20015, 26: 6643-6651; Rederstorff et al., Acta Biomaterialia, 2011, 7: 2119-2130; Rederstorff et al., Bone, 2011, 48: S141). Other approaches to induce chondrogenic differentiation in stem cell lines include appropriate chondrogenic differentiation media. Jennifer L. Puetzer *et al.* have reviewed the different chondrogenic differentiation media proposed to date by the scientific community (Tissue Engineering: Part B, Volume 16, Number 4, 2010). These authors conclude that none of these chondrogenic differentiation media is able to produce stable chondrocytes.

There is thus a need to provide new chondrogenic differentiation media having improved chondrogenic differentiation properties.

**Summary of the Invention**

The inventors have shown that the chondrogenic differentiation properties of chondrogenic differentiation media comprising growth factors known to induce chondrogenic differentiation can be dramatically increased by supplementing said media with specific low-molecular-weight sulfated polysaccharide derivatives of marine native exopolysaccharides (EPS).

The invention thus relates to chondrogenic differentiation media comprising:

- at least one chondrogenic growth factor selected from the group consisting of transforming growth factors β, bone morphogenetic proteins, and mixtures thereof, and
- a low-molecular-weight sulfated polysaccharide prepared from a marine native exopolysaccharide (EPS) excreted by a mesophilic marine bacterium from a deep-sea hydrothermal environment,

wherein said low-molecular-weight sulfated polysaccharide is obtainable by a process comprising the following steps:

(a) a step consisting of free-radical depolymerization of said native EPS so as to obtain a depolymerized EPS having a molecular weight of 5,000 to 100,000 g/mol,
(b) a subsequent step consisting of sulfation of the depolymerized EPS, comprising adding to the depolymerized EPS at least one sulfation agent in an amount sufficient to obtain a sulfated polysaccharide having a degree of sulfate-group substitution of between 10% and 45% by weight relative to the total weight of the sulfated polysaccharide.

The invention also relates to kits consisting of:

- at least one chondrogenic growth factor selected from the group consisting of transforming growth factors β, bone morphogenetic proteins, and mixtures thereof,
- a low-molecular-weight sulfated polysaccharide according to the invention, and

- a liquid cell culture medium.

Also described herein is a method for inducing chondrogenic differentiation in pluripotent or multipotent cells, wherein said method comprises the step of culturing pluripotent or multipotent cells with a chondrogenic differentiation medium according to the invention.

Also described herein is a method for obtaining a cartilage tissue wherein said method comprises the step of culturing pluripotent or multipotent cells with a chondrogenic differentiation medium according to the invention.

Also described herein are cartilage tissues obtained using such a method, and the use of these cartilage tissues as grafts in methods of treatment of the human or animal body.

**Definitions**

In the context of the present invention, the term *"Chondrogenic differentiation media"* refers to liquid media capable of inducing chondrogenic differentiation in pluripotent or multipotent cells cultured in said media. A chondrogenic differentiation medium according to the invention typically contains, in addition to said chondrogenic growth factor(s) and said derivative(s), nutrients necessary to support the growth, proliferation, differentiation and survival of cells. Thus, appropriate chondrogenic differentiation media according to the invention typically comprise a minimal medium in which cells can grow, such as for example Dulbecco modified Eagle's minimal essential medium (DMEM), Ham's F-12 Medium, or mixtures thereof, supplemented with at least one of the followings: glucose, bovine serum albumine (BSA), BD™ ITS or ITS+ Universal Culture Supplements, insulin, transferrin, selenous acid, linoleic acid, hydrocortisone, inorganic phosphate, ascorbate-2-phosphate, ascorbate, sodium ascorbate, calcium chloride, thyroxine, proline, pyruvate, sodium pyruvate, pyridoxine hydrochloride, glutamax, non essential amino acids, L-glutamine, β-glycero phosphate, dexamethasone. Examples of appropriate media are disclosed by Jennifer L. Puetzer et al. in Tissue Engineering: Part B, Volume 16, Number 4, 2010.

The term *"chondrogenic differentiation",* as used herein, refers to the differentiation of pluripotent or multipotent cells into cells producing an extracellular matrix close to that of cartilage. The chondrogenic differentiation of pluripotent or multipotent cells is typically evaluated by the measurement of the pellet volume, cell morphology and matrix composition by hematoxylin-eosin-safran (HES) and alcian blue staining, as fully described by Merceron C. et al. in Am J Physiol Cell Pysiol, 2010, 298(2):p.355-64. HES staining allows to distinguish cell nuclei and cytoplasm in the cells constituting the pellets, thereby evidencing the presence or not of a structural organization of the cells within the pellet, a structural organization being indicative of a chondrogenic differentiation. HES staining further colors collagen fibers within the matrix, which is also indicative of chondrogenic differentiation. Alcian blue staining reveals the presence of sulfated glycosaminoglycan (GAG), which is another marker of chondrogenic differentiation. In addition, immunohistological detection of type II, IX and XI collagens as well as aggrecan could also be used as further markers of chondrogenic differentiation. The chondrogenic differentiation of pluripotent or multipotent cells is also evaluated by measuring the expression level in the cells of mRNA encoding for COL2A1 (collagen of type II), ACAN (aggrecan), SOX9 and COMP (cartilage oligomeric matrix protein), which are specific markers of chondrocytes.

The term *"at least one Chondrogenic growth factor",* as used herein, refers to one, two, three, four or more chondrogenic growth factor(s).

The term *"pluripotent cells",* as used herein, refers to undifferentiated cells which can give rise to a variety of different cell lineages of the three germ layers (endoderm, mesoderm and ectoderm). Typically, pluripotent cells may express the following markers oct4, SOX2, Nanog, SSEA 3 and 4, TRA 1/81, see International Stem Cell Initiative recommendations (Nature Biotechnology 25, 803 - 816 (2007)).

The terms "*multipotent cells"* and "*progenitor cells"* are used herein interchangeably. They refer to undifferentiated cells which can give rise to a limited number of different cell lineages, including chondrocytes. Typical multipotent cells according to the invention are mesenchymal stem cells.

As used herein, the term *"subject"* refers to a mammal, preferably a human being, that may or may not suffer from a disease associated with cartilage damage.

The term "*treating*" or *"treatment"* refers to a method that is aimed at delaying or preventing the onset of a disease, at reversing, alleviating, inhibiting, slowing down or stopping the progression, aggravation or deterioration of the symptoms of the disease, at bringing about ameliorations of the symptoms of the disease, and/or at curing the disease.

**Detailed Description of Certain Preferred Embodiments**

**I - Chondrogenic differentiation media**

The invention relates to chondrogenic differentiation media comprising:

- at least one chondrogenic growth factor selected from the group consisting of transforming growth factors β, bone morphogenetic proteins, and mixtures thereof, and
- a low-molecular-weight sulfated polysaccharide prepared from a marine native exopolysaccharide (EPS) excreted by a mesophilic marine bacterium from a deep-sea hydrothermal environment,

wherein said low-molecular-weight sulfated polysaccharide is obtainable by a process comprising the following steps:

(a) a step consisting of free-radical depolymerization of said native EPS so as to obtain a depolymerized EPS having a molecular weight of 5,000 to 100,000 g/mol,
(b) a subsequent step consisting of sulfation of the depolymerized EPS, comprising adding to the depolymerized EPS at least one sulfation agent in an amount sufficient to obtain a sulfated polysaccharide having a degree of sulfate-group substitution of between 10% and 45% by weight relative to the total weight of the sulfated polysaccharide derivative.

### 1. Processes for obtaining low-molecular-weight sulfated polysaccharides

Processes for obtaining low-molecular-weight sulfated polysaccharide derivatives of marine native exopolysaccharides (EPS) according to the invention are fully described in the international application WO 2006/003290, and also by Colliec Jouault S. et al. in Biochim Biophys Acta 2001, 1528(2-3):p.141-151, and by Guezenec J. et al. in Carbohydrate Polymers 1998, 37(1):p.19-24.

In certain embodiments, the depolymerized derivatives obtained after step (a) are lyophilized.

In other embodiments, step (b) of the process is followed by a dialysis step.

During the first depolymerization step, the native EPS can be used in a liquid form, *i.e.* as it is excreted by the bacteria into the culture medium. Preferably, the culture medium is centrifuged and only the supernatant containing the native EPS and that is free of bacterial debris is collected. The native EPS can be collected by any suitable technique known to those skilled in the art, such as for example membrane ultrafiltration, and can then optionally be lyophilized as is or in the form of an addition salt.

The step consisting of free-radical depolymerization of the native EPS is preferably carried out by addition of a solution of an oxidizing agent to a reaction mixture comprising the native EPS, preferably in the presence of a metal catalyst. The oxidizing agent is preferably chosen from peroxides, in particular hydrogen peroxide, and peracids, in particular peracetic acid and 3-chloroperbenzoic acid. The addition is preferably carried out continuously and with stirring for a period of between 30 minutes and 10 hours. Reaction mixture is preferably maintained at a pH of between 6 and 8, for example by addition of a basifying agent such as sodium hydroxide, and at a temperature of between approximately 30°C and 70°C throughout the duration of the free-radical depolymerization reaction.

According to a specific embodiment of the present invention, in this step, the native EPS is present in the reaction mixture at a concentration of between approximately 2 and 10 mg/ml of reaction mixture.

In preferred embodiments, the oxidizing agent is a solution of hydrogen peroxide ($H_2O_2$) preferably having a concentration of between approximately 0.1% and 0.5% by weight, preferably of the order of 0.1% to 0.2% by weight, and is added at a flow rate of V1/1000 to V1/10 ml/minute, preferably V1/50 and V1/500 ml/minute, and more preferably of the order of V1/100 ml/minute, V1 being the volume of the reaction medium containing a marine exopolysaccharide (EPS) to which a solution of hydrogen peroxide is added.

The metal catalysts that can be used during the depolymerization step are preferably chosen from $Cu^{++}$, $Fe^{++}$ and $Cr^{+++}$ ions and the $Cr_2O_7^{2-}$ anion, as described in particular in patent application EP 0 221 977. According to a specific embodiment, the metal catalyst is present in the reaction mixture at a concentration of between approximately $10^{-3}$ M and $10^{-1}$ M, and preferably at a concentration of between approximately 0.001 and 0.05 M.

The free-radical depolymerization process in accordance with the invention and as described above makes it possible to obtain, in a single step and with a good yield, homogeneous, low-molecular-weight polysaccharide derivatives. In the context of the present invention, the term "homogeneous derivatives" is intended to mean derivatives which, when assessed using high performance size exclusion chromatography, exhibit a single main peak representing a predominant population of polysaccharide chains that are homogeneous with respect to size, characterized by a polydispersity index I (Mw/Mn) < 5, where Mw = weight-average molecular weight and Mn = number-average molecular weight.

In certain embodiments, when the depolymerization reaction is over, the polysaccharide derivatives obtained are reduced using a reducing agent, so as to stabilize the chains, the reducing ends of which are very reactive, and in particular to avoid chain hydrolysis by the "peeling" reaction. The nature of the reducing agents that can be used to this effect is not essential. The reducing agent may be, in particular, sodium borohydride.

The metal catalyst used in the depolymerization step can be eliminated at the end of the depolymerization reaction, (or at the end of the reduction reaction if a reduction step is carried out) using any suitable method, for example by ion exchange chromatography, preferably a weak cation exchange resin passivated beforehand, or by treatment with EDTA (ethylenediaminetetraacetic acid).

In certain embodiments, prior to the sulfation step, a step consisting of N-deacetylation of the polysaccharide derivatives comprising N-acetylated hexosamines which are obtained at the end of the free-radical depolymerization step and/or at the end of the reduction step is carried out. This N-deacetylation step is carried out according to a protocol adapted from Z ou et al. (Carbohyd. Res., 1998, 309: 297-301). Advantageously, the N-deacetylation step is carried out by addition, to the reaction mixture comprising the polysaccharide derivatives, of a solution of sodium borohydride, under stirring conditions. When the temperature of the reaction mixture reaches approximately 80°C, a basifying agent, preferably sodium hydroxide, is added to the reaction medium. After reaction for one hour, the reaction medium is neutralized by continuous addition of acetic acid until a pH of 5 is obtained. The polysaccharide derivatives obtained can be recovered by membrane ultrafiltration and then can optionally be lyophilized.

The polysaccharide derivatives resulting from the depolymerization and/or from the reduction and/or from the N-deacetylation can, if necessary, be recovered by any suitable technique well known to those skilled in the art, such as, for example, by membrane ultrafiltration or dialysis. Then, they are lyophilized and fractionated by size exclusion chromatography to increase their purity required to improve the subsequent sulfation step. Finally, the purified polysaccharide derivatives are conditioned in salt form by addition of a weak or strong base, that may be chosen, for example, from pyridine, triethylamine, tributylamine, tetrabutylammonium hydroxide and sodium hydroxide. This lyophilized salt may be prepared, for example, by elution of an aqueous solution of the polysaccharide derivatives at a concentration of between 1 and 8 mg/ml on an ion exchange resin column such as, for example, those sold under the name Dowex® by the company Dow Chemical. The eluate is collected as long as the pH remains acid, for example less than 5, then the pH is subsequently adjusted to approximately 6.5 with the desired base as defined above. The polysaccharide derivatives in the form of a salt are then ultrafiltered and lyophilized.

The lyophilized polysaccharide derivatives, possibly in the form of an addition salt, are preferably dissolved in an anhydrous solvent at the beginning of the sulfation step; this solvent is preferably chosen from dimethylformamide (DMF), dimethyl sulfoxide (DMSO) formamide, and mixtures thereof. The amount of polysaccharide derivatives present in the anhydrous solvent may be between approximately 1 and 10 mg/ml, preferably between approximately 1 and 5 mg/ml, and even more preferably this amount is approximately 2.5 mg/ml. The dissolution of the EPS in the anhydrous solvent is preferably carried out, with stirring, at ambient temperature for approximately 1 to 2 hours and then at a temperature of between 40°C and 50°C, preferably at a temperature of approximately 45 °C for approximately 2 hours under argon or azote with molecular sieve.

The one or more chemical sulfation agents used during the sulfation step can be added to the depolymerized and/or reduced and/or N-deacetylated EPSs that are in lyophilized form or in the form of a solution.

The sulfation agents are preferably chosen from complexes of pyridine sulfate (free or coupled to a polymer), of dimethylformamide sulfate, of triethylamine sulfate and of trimethylamine sulfate. The one or more chemical sulfation agents are added to the solution of polysaccharide derivatives in a weight amount preferably representing from approximately 4 to 6 times, and even more preferably approximately 5 times, the mass of polysaccharide derivatives in solution. The chemical sulfation reaction is then preferably carried out with stirring for a period of between approximately 2 and 24 hours depending on the desired degree of sulfation. When the desired degree of sulfation is reached, the sulfation reaction is stopped after cooling of the reaction medium:

- either by precipitation in the presence of sodium-chloride-saturated acetone or of methanol, and then dissolution of the precipitate in water;
- or, preferably, by addition of water in a proportion preferably equal to 1/10 of the reaction volume and adjustment of the pH of the reaction medium to 9 with a basifying agent such as, for example, sodium hydroxide (3 M).

According to certain embodiments, the solution of sulfated polysaccharide derivatives is preferably dialyzed in order to remove the various salts, and then lyophilized. The final product, typically with an accurate molecular weight and a low polydispersity index of less than 2, is obtained by fractionation performed by size exclusion chromatography.

The low-molecular-weight sulfated polysaccharide derivatives in accordance with the invention have a molecular weight of 5,000 to 100,000 g/mol, 5,000 to 60,000 g/mol, 5,000 to 50,000 g/mol, 5,000 to 40,000 g/mol, preferably of 5,000 to 30,000 g/mol, more preferably of 10,000 to 25,000 g/mol.

The low-molecular-weight sulfated polysaccharide derivatives in accordance with the invention have a polydispersity index of less than 5, preferably of 1.5 to 4, more preferably of less than 2. The polydispersity index (PDI) according to the invention is a measure of the distribution of molecular mass of the derivatives. The PDI calculated is the weight average molecular weight divided by the number average molecular weight. PDI is typically measured by size-exclusion chromatography.

The sulfated polysaccharide derivatives according to the invention have a degree of sulfate-group substitution of between 10% and 45% by weight relative to the total weight of the sulfated polysaccharide derivative. In certain embodiments, the degree of sulfate-group substitution is of between 10% and 40%, of between 20% and 45% or of between 20% and 40%.

In certain embodiments, the sulfated polysaccharide derivatives according to the invention have a molecular weight of 5,000 to 50,000 g/mol, a polydispersity index of less than 5, particularly of 1.5 to 4, more particularly of less than 2, and a degree of sulfate-group substitution of between 20% and 40% by weight relative to the total weight of the sulfated polysaccharide derivative.

### 2. Mesophilic marine bacteria

The derivatives according to the invention are prepared from marine native exopolysaccharides (EPS) excreted by a mesophilic marine bacterium from a deep-sea hydrothermal environment selected from the group consisting of bacteria of the *Alteromonas* genus, *Pseudoalteromonas* genus and *Vibrio* genus. In a particular embodiment, the bacterium of the *Alteromonas* genus is selected from the group consisting of the strains GY785, HYD 657, HYD 708, HYD 721, HYD 1545, HYD 1644, ST 716 and MS 907. In another particular embodiment, the mesophilic marine bacterium is the strain HE 800 of the *Vibrio* genus.

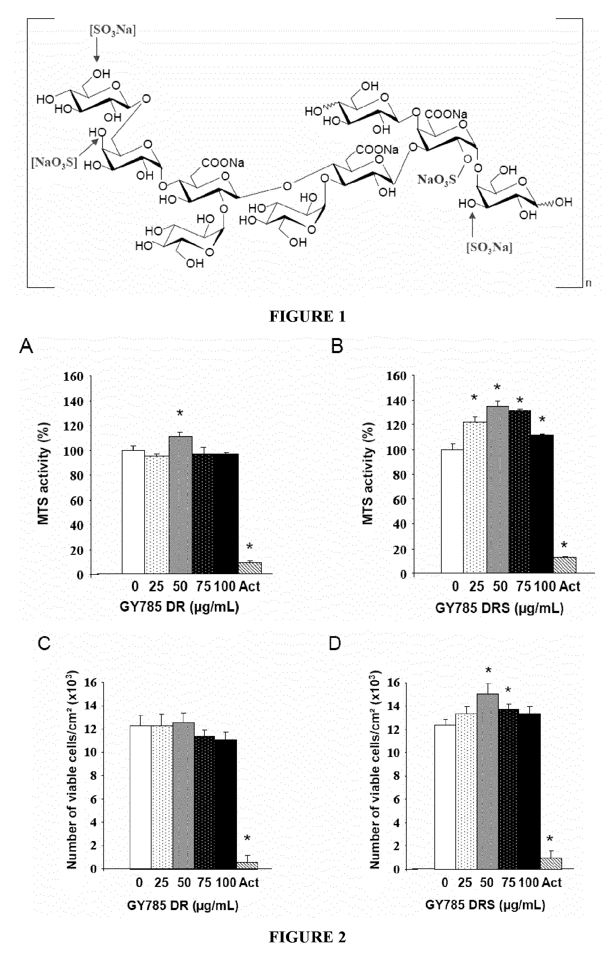

In certain preferred embodiments, the derivative according to the invention is prepared from the strain GY785 of the *Alteromonas* genus (*Alteromonas infernus*). Such a derivative is called "GY 785 DRS" (see figure 1 for molecular structure).

### 3. Chemical characteristics of the native EPSs and derivatives

In certain embodiments, the low-molecular-weight sulfated polysaccharide derivatives are obtained from native EPSs excreted by bacteria of the *Alteromonas* genus. The native EPSs typically have a neutral monosaccharide content of from 20% to 70%, preferably from 30% to 60%, and more preferably from 38% to 57% by weight. In addition, the native EPSs typically have an acidic monosaccharide content of from 5% to 60%, preferably of between 6% and 50%, and more preferably of between 8% and 42% by weight. The native EPSs also typically have an amino sugar content of from 0% to 1% by weight in their oside composition.

In a particular embodiment, the native EPSs excreted by bacteria of the *Alteromonas* genus have an oside composition comprising:

- from 20% to 70%, preferably from 30% to 60%, and more preferably from 38% to 57% by weight of neutral monosaccharides,
- from 5% to 60%, preferably from 6% to 50%, and more preferably from 8% to 42% by weight of acidic monosaccharides,
- from 0% to 1% by weight of amino sugars.

According to another embodiment, the low-molecular-weight sulfated polysaccharide of the invention are obtained from native EPSs excreted by bacteria of the *Vibrio* genus, preferably by the bacterial strain HE 800. The native EPSs excreted by bacteria of the *Vibrio* genus are not sulfated. The native EPSs typically have a neutral monosaccharide content of from 0% to 5%, preferably from 0% to 1% by weight. The native EPSs also typically have an acidic monosaccharide content of from 20% to 50%, preferably from 25% to 40%, and more preferably from 30% to 32% by weight. The native EPSs still typically have an amino sugar content of from 20% to 50%, preferably from 25% to 40%, and more preferably from 30% to 35% by weight. Lastly, the native EPSs typically have an N-acetylated group content of from 0% to 15%, preferably from 4% to 8%, and more preferably from 5% to 6% by weight.

In a particular embodiment, the native EPSs excreted by bacteria of the *Vibrio* genus have an oside composition comprising:

- from 0% to 5%, preferably from 0% to 1% by weight of neutral monosaccharides,
- from 20% to 50%, preferably from 25% to 40%, and more preferably from 30% to 32% by weight of acidic monosaccharides,
- from 20% to 50%, preferably from 25% to 40%, and more preferably from 30% to 35% by weight of amino sugars,
- from 0% to 15%, preferably from 4% to 8%, and more preferably from 5% to 6% by weight of N-acetylated groups.

Still typically, the native EPSs excreted by a mesophilic marine bacterium according to the invention have a protein content of from 0% to 15%, preferably from 0% to 5%, and more preferably from 0% to 1% by weight.

### 4. Amount of derivatives in the chondrogenic differentiation media

In certain embodiments, chondrogenic differentiation media according to the invention comprise 5μg/mL to

200μg/mL, particularly 25μg/mL to 100μg/mL, more particularly 25μg/mL to 75μg/mL, most particularly about 50μg/mL of said derivative.

### 5. Chondrogenic growth factors

The chondrogenic growth factors are fully described in the prior art and well known by the skilled person. The review by Jennifer L. Puetzer (Tissue Engineering: Part B, Volume 16, Number 4, 2010) for instance discloses different chondrogenic growth factors, used alone or in combination to induce chondrogenic differentiation of pluripotent or multipotent cells.

Major chondrogenic growth factors families are transforming growth factors β and bone morphogenetic proteins. The transforming growth factors β are typically selected from TGF-β1, TGF-β2 and TGF-β3. The bone morphogenetic proteins are typically selected from BMP-2, BMP-4, BMP-6, BMP-7 and BMP-9.

Other chondrogenic growth factors include fibroblast growth factors, in particular FGF-2, and insulin-like growth factors, in particular IGF-1.

Examples of particular chondrogenic growth factor or mixtures of chondrogenic growth factors that may be included in chondrogenic differentiation media according to the invention are: TGF-β1; TGF-β2; TGF-β3; TGF-β1 + TGF-β2 + TGF-β3; TGF-β1 + FGF-2; TGF-β1 + IGF-1; TGF-β1 + BMP-2; TGF-β1 + BMP-6; TGF-β2 + IGF-1; TGF-β3 + FGF-2; BMP-2; BMP-4; BMP-6; BMP-7; BMP-9; BMP-2 + BMP-9; TGF-β3 + BMP-6; TGF-β3 + BMP-2; TGF-β3 + BMP-6 + IGF-1; TGF-β1 + TGF-β3 + BMP-6 + IGF-1; BMP-2 + BMP-7; TGF-β3 + BMP-2 + BMP-4 + BMP-6 + BMP-7 + IGF-1; TGF-β2 + BMP-2 + BMP-6 + BMP-7.

In certain embodiments, the media according to the invention typically also comprise insulin, transferrin, selenous acid or ITS or ITS+ Universal Culture Supplements (BD™).

### II - Kits for chondrogenic differentiation

The invention also relates to kits consisting of at least one chondrogenic growth factor selected from the group consisting of transforming growth factors β, bone morphogenetic proteins, and mixtures thereof, a low-molecular-weight sulfated polysaccharide prepared from a marine native exopolysaccharide (EPS) excreted by a mesophilic marine bacterium from a deep hydrothermal environment, as described above, and a liquid cell culture medium.

Examples of liquid cell culture media typically comprise a minimal medium in which cells can grow, such as for example Dulbecco modified Eagle's minimal essential medium (DMEM), Ham's F-12 Medium, or mixtures thereof, supplemented with at least one of: glucose, bovine serum albumine (BSA), BD™ ITS or ITS+ Universal Culture Supplements, insulin, transferrin, selenous acid, linoleic acid, hydrocortisone, inorganic phosphate, ascorbate-2-phosphate, ascorbate, sodium ascorbate, calcium chloride, thyroxine, proline, pyruvate, sodium pyruvate, pyridoxine hydrochloride, glutamax, non essential amino acids, L-glutamine, β-glycero phosphate, dexamethasone. Particular examples of suitable cell culture media are fully described in the review by Jennifer L. Puetzer (Tissue Engineering: Part B, Volume 16, Number 4,2010).

### III - Chondrogenic differentiation

*Ex vivo* methods for inducing chondrogenic differentiation in pluripotent or multipotent cells are described, wherein the methods comprise a step of culturing pluripotent or multipotent cells with a liquid chondrogenic differentiation medium according to the invention.

*Ex vivo* methods for obtaining a cartilage tissue are described, wherein the methods comprise a step of culturing pluripotent or multipotent cells with a liquid chondrogenic differentiation medium according to the invention.

The cartilage tissue obtained using such a method is also described.

Also described is the cartilage tissue for use in methods of treatment of the human or animal body, in particular for use in methods of treatment of a disease associated with cartilage damage in the human or animal body.

A treatment of a disease associated with cartilage damage in a subject in need thereof, comprises a step of placing in said subject a cartilage tissue. The step of placing of such a cartilage tissue in a subject may be carried out by grafting.

Examples of diseases associated with cartilage damage include, but are not limited to, osteoarthritis, traumatic rupture or detachment of cartilage, osteochondritis, degenerative disc disease (degeneration of the intervertebral disc), relapsing polychondritis.

The step of culturing pluripotent or multipotent cells with a liquid chondrogenic differentiation medium of the invention must be carried out for a time duration allowing chondrogenic differentiation. Typically, the culture of pluripotent or multipotent cells with the medium of the invention is carried out for at least 15 days, preferably at least 20 days, even more preferably at least 25 days, most preferably during 28 days.

If necessary, the liquid chondrogenic differentiation medium of the invention can be renewed, partly or totally, at regular intervals. Typically, the chondrogenic differentiation medium of the invention can be replaced with fresh chondrogenic differentiation medium of the invention every 2-3 days, for 28 days.

The culture of human stem cells in the liquid chondrogenic differentiation medium may be performed by using a three-dimensional culture system. Three-dimensional culture systems are particularly preferred for obtaining cartilage tissues. Examples of three-dimensional culture systems are pellets, micromass, high density cell cultures, and tridimensional cultures in biomaterials, fully described by Vinatier C. et al. in Trends Biotechnol, 2009, 27(5): p.307-14.

The pluripotent or multipotent cells may be human pluripotent or multipotent cells.

The pluripotent or multipotent cells may be non-human mammalian pluripotent or multipotent cells.

The pluripotent or multipotent cells may be stem cells.

The pluripotent or multipotent cells may be multipotent stem cells, particularly human mesenchymal stem cells. The human mesenchymal stem cells are typically isolated from bone marrow, adipose tissue, synovial membrane, umbilical cord blood, muscle, periosteum or placenta. The human mesenchymal stem cells may be human adipose tissue-derived stem cells (hATSC).

The pluripotent or multipotent cells may be pluripotent cells, in particular embryonic stem cells.

The pluripotent cells may be human embryonic stem cells (hES cells). Typically, hES cell lines such as the ones described in the table below may be employed:

| line | karyotype | passage available | country of origin | origin |
|---|---|---|---|---|
| SA01 | 46XY | 25 | Sweden | Cellartis AB |
| VUB01 | 46XY | 73 | Belgium | AZ-VUB Bruxel |
| HUES 24 | 46XY | 26 | USA | Harvard |
| H1 | 46XY, 20q11.21 | 26 | USA | Wicell research Institute |
| H9 | 46XX | 27 | USA | Wicell research Institute |
| WT3 | 46XY | 35 | UK | UKSCB |

The pluripotent cells may be non-human embryonic stem cells, such a mouse stem cells.

The pluripotent cells may be induced pluripotent stem cells (iPS). Induced pluripotent stem cells (iPS cells) are a type of pluripotent stem cells artificially derived from a non-pluripotent, typically an adult somatic cell, by inducing a "forced" expression of certain genes. iPS cells were first produced in 2006 from mouse cells (Takahashi et al. Cell 2006 126 :663-76) and in 2007 from human cells (Takahashi et al. Cell 2007 131-861-72, Yu et al. Science 2007 318 :1917).

Further aspects and advantages of this invention will be disclosed in the following figures and examples, which should be regarded as illustrative.

## Brief Description of the Figures

**Figure 1:** Structure of the nonasaccharidic repeating unit of GY785 DR. GY785 DR is naturally sulfated at position C2 of the galacturonic acid residue in blue. GY785 DRS is a chemically over-sulfated form of GY785 DR. Putative positions for additional sulfate groups ($SO_3Na$) are indicated by arrows.

**Figure 2:** Human ATSC viability and proliferation. Human ATSC were cultured in the presence of either GY785 DR (A and C), GY785 DRS (B and D) at the indicated concentrations of actinomycin D (5$\mu$g/mL) for 72 hours. Viability (A and B) was evaluated by MTS activity measurement and expressed as the relative MTS activity compared to the untreated control. Proliferation (C and D) was estimated by viable cell counting after trypan blue exclusion dye. * $P<0.05$ compared to the control condition (0$\mu$g/mL of polysaccharide).

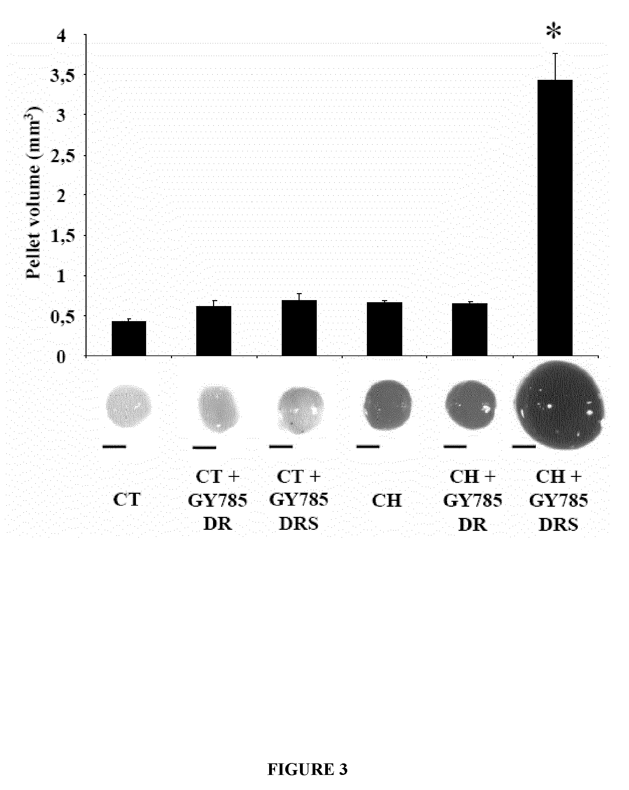

**Figure 3:** Histological characterization of hATSC pellets. Human ATSC were cultured in pellets in the presence of control (CT) or chondrogenic (CH) medium supplemented or not with 50$\mu$g/mL of GY785 DR or GY785 DRS during 28 days. The pellet volumes were calculated by equating these entities to ellipsoids. * $P<0.05$ compared to the control condition (0$\mu$g/mL of polysaccharide). GAG production was evaluated at a gross level after alcian blue staining of the whole pellets. Bar: 500$\mu$m.

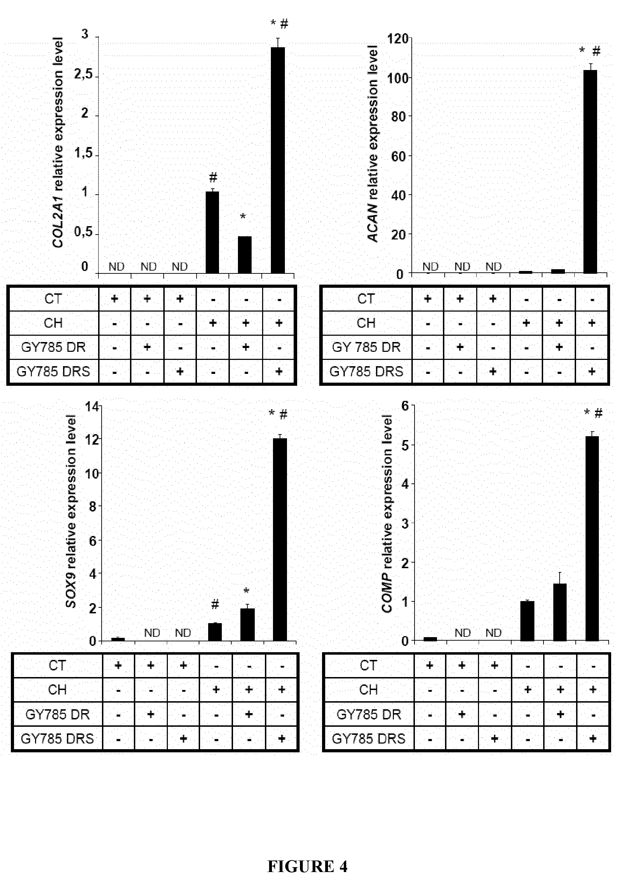

**Figure 4:** Analysis of the expression levels of chondrogenic markers. Human ATSC pellets were cultured in the presence of control (CT) or chondrogenic (CH) medium supplemented or not with 50$\mu$g/mL of GY785 DR or GY785 DRS for 28 days. Expression of the chondrogenic markers *COL2A1, ACAN, SOX9* and *COMP* was investigated by real-time PCR. Results are expressed as relative expression level compared to the chondrogenic medium in the absence of polysaccharide. * *p* <0.05 compared to chondrogenic medium (CH). # *p* <0.05 compared to chondrogenic medium supplemented with GY785 DR.

## Examples

## Materials and Methods

### *Materials*

Cell culture plasticwares were purchased from Corning-Costar BV Life Sciences (Schipol-Rijk, The Netherlands). Hank's Balanced Sodium Salt (HBSS), Dulbecco's Modified Eagle Medium high glucose (4.5g/L) (DMEM), alpha minimum essential medium ($\alpha$MEM), phosphate buffered salt (PBS), penicillin/streptomycin, trypsin/EDTA (0.05%/0.53mM), L-glutamine, superscript III kit, NuPAGE™ 4%-12% Bis-Tris gel, PVDF (polyvinylidene difluoride). Invitrolon membranes were obtained from Invitrogen Corporation (Paisley, UK). MTS reagents were from Promega (Charbonnières, France). Collagenase crude type I A, red blood cell lysis buffer, trypan blue, sodium L-ascorbate, ITS media supplement, dexamethasone, alcian blue and anisomycin were purchased from Sigma-Aldrich (St. Louis, MO). Brilliant® SYBR® Green Master Mix was obtained from Stratagene Europe (Amsterdam Zuidoost, The Netherlands). PCR primers were synthesized by MWG Biotech (Ebersberg, Germany). Fetal calf serum (FCS) was purchased from Dominique Dutscher (Brumath, France). TGF-β1 was obtained from PeproTech Inc. (London, UK). RNeasy micro kit was purchased from Qiagen and turbo DNase from Ambion Inc. both distributed by Applied Biosystem (Courtaboeuf, France). Protein content was determined using the Pierce Coomassie Plus assay (Pierce, Rockford, IL). The rabbit anti phospho-SMAD 2 (3101), phospho-ERK1/2 (9101), phospho-JNK 1/2 (9251), phospho-p38 (9211), SMAD 2 (3102), ERK1/2 (9102) and goat anti-rabbit IgG HRP-linked (7074) antibodies were purchased from Cell Signaling Inc. (Beverly, MA). The Western blot detection system was obtained from GE Healthcare (Buckinghamshire, UK). All the other chemicals were obtained from standard laboratory suppliers and were of the highest purity available.

### *Production, purification and characterization of GY785 DR and GY785 DRS polysaccharide*

The two low molecular weight polysaccharides GY785 DR and GY785 DRS were derived from GY785, a high molecular weight exopolysaccharide ($1.5 \times 10^6$ Da) produced by a bacterium: *Alteromonas infernus*, isolated from a sample of fluid collected in the vicinity of an active hydrothermal vent. The isolation procedure and characterization of the strain *Alteromonas infernus* have previously been described by Raguenes *et al.* (Raguenes GH et al. J Appl Microbiol. 1997;82:422-430). The native polysaccharide can undergo a radical depolymerization to obtain GY785 DR. Then, the GY785 DR can be chemically over-sulfated to obtain the GY785 DRS as described elsewhere (Guezennec J et al. Carbohydrate Polymers. 1998;37:19-24). GY785 DR and GY785 DRS are homogenous fractions with average molecular mass of 15 kDa and 20 kDa and 10% and 45% sulfate groups, as determined by analytical high performance size-exclusion chromatography and elemental analysis, respectively (Figure 1) (Colliec Jouault S et al. Biochim Biophys Acta. 2001;1528:141-151).

### *Cell culture*

Human adipose tissue stromal cells (hATSC) were isolated by collagenase digestion of lipoaspirates obtained from 3 different patients undergoing liposuction and who had given their informed consent. All protocols were approved by the French national ethical committee. Briefly and as previously described (Estes BT et al. Nat Protoc. 2010;5:1294-1311; Merceron C et al. Am J Physiol Cell Physiol. 2010;298:355-364), lipoaspirates were washed extensively with HBSS to remove debris. Washed lipoaspirates were treated with collagenase (0.025%) in HBSS for 1 hour at 37°C with gentle agitation. The collagenase treatment was inactivated by adding an equal volume of DMEM high glucose containing 1% penicillin/streptomycin, 1% L-glutamine and 10% FCS (control medium). The digested product was then centrifuged at 250 x g for 5 min to separate adipocytes from stromal cells. The supernatant was removed and cells were re-suspended in the control medium and filtered through a 70$\mu$m nylon mesh filter. The filtrate was centrifuged and cells re-suspended in red blood cell lysis buffer. The lysis reaction was stopped by adding control medium. The suspension was centrifuged and cells were finally re-suspended in control medium and plated at $5 \times 10^4$ cells/cm$^2$ in 75cm$^2$ culture flasks. Cells were incubated at 37°C in a humidified atmosphere containing 5%CO$_2$ and 95% air and the control medium was replaced 24 hours after seeding to remove non adherent cells. Thereafter, the control medium was renewed every 2-3 days. To prevent spontaneous differentiation, hATSC primary cultures were grown to 90% confluence and then detached from the cell culture flask using trypsin/EDTA and plated at $10^4$ cells/cm$^2$. For all subsequent experiments hATSC were used at passage 2. Human ATSC isolated using the above described protocol have been extensively characterized in our laboratory (for details see Merceron C et al. Am J Physiol Cell Physiol. 2010;298:355-364, and Merceron C et al. The effect of two and three dimensional cell culture on the chondrogenic potential of human adipose-derived mesenchymal stem cells after subcutaneous transplantation with an injectable hydrogel, Cell Transplant. 2011 Feb 3., Epub ahead of print).

**Viability and proliferation**

Cell viability was evaluated using a MTS assay as previously described (Vinatier C et al. Biomaterials. 2005;26:6643-6651). hATSC were plated in 24-well plates at a density of $1 \times 10^4$ cells/$cm^2$ and cultured in control medium in the absence or presence of 25, 50, 75 and 100 $\mu$g/mL of GY785 DR or GY785 DRS for 72 hours. As a negative control, cells were cultured in the presence of actinomycin D (5$\mu$g/mL) a well known cell death inducer (Vinatier C et al. Biomaterials. 2005;26:6643-6651). Briefly, culture medium was removed and replaced by fresh medium containing MTS reagents according to the manufacturer's instructions. Results were expressed as relative MTS activity compared to the control condition (cells cultured in the absence of polysaccharide).

To correlate MTS activity with cell proliferation, the number of viable cells was also estimated. As previously described, cells were treated with trypsin/EDTA after 72h of culture and counted using trypan blue exclusion dye (Merceron C et al. Am J Physiol Cell Physiol. 2010;298:355-364; Vinatier C et al. Biomaterials. 2005;26:6643-6651). Results were expressed as the total number of viable cells/$cm^2$ compared with the control condition (cells cultured in the absence of polysaccharide).

**Chondrogenic differentiation**

For the *in vitro* chondrogenic differentiation of hATSC, $5 \times 10^5$ cells were placed into a 15 mL polypropylene tube containing 1mL of control medium, as previously described (Merceron C et al. Am J Physiol Cell Physiol. 2010;298:355-364). They were then centrifuged for 5 min at 250 x g. The tubes were fitted with vented caps to permit gas exchange, and the cell pellets were maintained at 37°C in a humidified atmosphere containing 5% $CO_2$ and 95% air. After 24 hours, pellets of hATSC were divided into 6 experimental groups and cultured either in the presence of control (CT) or chondrogenic (CH) medium alone or supplemented with GY785 DR or GY785 DRS. The chondrogenic medium was composed of serum-free control medium supplemented with 6.25$\mu$g/mL insulin, 6.25$\mu$g/mL transferin, 6.25ng/mL sodium selenite (ITS), 50nM sodium L-ascorbate, $10^{-8}$M dexamethasone and 10ng/mL TGF-$\beta$1 (Merceron C et al. Am J Physiol Cell Physiol. 2010;298:355-364; Merceron C et al. The effect of two and three dimensional cell Culture on the chondrogenic potential of human adipose-derived mesenchymal stem cells after subcutaneous transplantation with an injectable hydrogel, Cell Transplant. 2011 Feb 3., Epub ahead of print). Culture media were changed every 2-3 days for 28 days. Pellets were maintained at 37°C in a humidified atmosphere (5% $CO_2$ and 95% air).

**Human ATSC pellet characterization**

For the measurement of the pellet volume, pellets were considered as scalene ellipsoid entities. Their volumes were estimated using the following formula:

$$\text{Scalene ellipsoid volume} = 4/3\ \pi\ a\ b\ c$$

where a, b and c represent the radius of long axis in each spatial plane.

For pellet gross appearance, the production of sulfated GAGs was investigated on whole 28-day old pellets by alcian blue staining. Pellets were washed with ice-cold PBS and fixed for 20 minutes in 100% ethanol. Pellets were then stained at room temperature with 0.1% alcian blue solution in 0.1 M HCl. After overnight incubation, the solution was discarded and the pellets were rinsed with 0.1 M HCl to eliminate nonspecific staining. Photographs were obtained with a stereo-microscope (Leica MZ6, Wetzlar, Germany).

For histological analysis, 28 day-old pellets of hATSC were fixed in 10% formalin and embedded in paraffin. Paraffin sections (5 $\mu$m thick) were de-paraffinized using toluene, rehydrated through a graded series of ethanol, and rinsed in distilled water. Tissue sections were stained with hematoxylin-eosin-safran (HES) and alcian blue as previously described (Merceron C et al. Am J Physiol Cell Physiol. 2010;298:355-364). Sections were then visualized using a light microscope (Zeiss Axioplan 2, Göttingen, Germany). Alcian blue reveals the presence of a GAG containing cartilaginous matrix and HES stains nucleus in purple, cytoplasm in pink and collagen fibres in yellow.

*Transcript analysis*

Total RNA from 28 day-old pellets of hATSC was extracted using the RNeasy micro kit in accordance with the manufacturer's instructions. After DNase digestion, RNA was quantified using a UV-spectrophotometer (Nanodrop ND-1000, Labtech, Palaiseau, France) and quality was determined with the Agilent Bioanalyser 2100 system (Waldbronn, Germany). 500ng of RNA per sample were reverse transcribed using the superscript III kit in a total volume of 30μL. Complementary DNA (cDNA) was amplified in a total volume of 25 μL PCR reaction mix containing 12.5 μL of Brilliant® SYBR® Green Master Mix (1X) and 30nM of SYBR green reference dye. The sequence and concentration of each primer set are provided in Table 1.

Table 1: Sequences of primer pairs, gene bank accession numbers used for real time RT-PCR analysis and size of PCR products.

| Gene | Gene Bank Accession Number | Sequence | Base Pairs (bp) |
|---|---|---|---|
| β-*ACTIN* | NM_001101 | Fwd 5'- CCAACCGCGAGAAGATGA -3' Rev 5'- CCAGAGGCGTACAGGGATAG -3' | 97 |
| *Type II collagen - α1 chain (COL2A1)* | NM_001844 | Fwd 5'- TGTCAGGGCCAGGATGTC -3' Rev 5'- ATCATTATACCTCTGCCCATCC -3' | 63 |
| *Aggrecan (ACAN)* | NM_001135 | Fwd 5'- CCTCCCCTTCACGTGTAAAA -3' Rev 5'- GCTCCGCTTCTGTAGTCTGC -3' | 64 |
| *Sex determining region Y-box 9 (SOX9)* | NM_000346 | Fwd 5'- GTACCCGCACTTGCACAAC -3' Rev 5'- TCGCTCTCGTTCAGAAGTCTC -3' | 72 |
| *Cartilage Oligomeric Matrix Protein (COMP)* | NM_000095 | Fwd 5'- GCACCGACGTCAACGAGT -3' Rev 5'- TGGTGTTGATACAGCGGACT -3' | 63 |

The real-time polymerase chain reaction was carried out in a MX3000P® real-time PCR system (Stratagene) under the following conditions: 10min at 95°C followed by 40 cycles of 30 s at 95°C, 1 min at 60°C and 30 s at 72°C. The efficiency and specificity of each primer set was confirmed with standard curves of cycle threshold (Ct) values versus serial dilution of total RNA and melting profile evaluation. Cycle Thresholds were normalized to β-actin to control for cDNA quantification differences. Results were reported as relative expression levels compared to the cells cultured in the presence of chondrogenic medium.

*Surface plasmon resonance*

Experiments were carried out on a Biacore 3000 instrument (Biacore, Uppsala, Sweden). TGF-β1 and insulin were covalently immobilized to the dextran matrix of a CM5 sensor chip (Biacore) as recommended by the manufacturer at flow rate of 5 μL/min. Binding assay of GY785 DR (1.5, 3.125, 6.25, 12.5, 25, 50 and 100 μg/mL) and GY785 DRS (0.0312, 0.0625, 0.125, 0.25, 0.50 and 1 μg/mL) were performed in 10mM HEPES buffer, pH 7.4, containing 0.15M NaCl and 0.005% P20 surfactant (HBS-P buffer,Biacore) and dissociation was monitored for 15 minutes. Regeneration was achieved with NaOH (4.5 mmol/L) after each cycle. The resulting sensorgrams were fitted using BiaEval 4.1 software (Biacore). For $K_d$ calculations, the following molecular weights were used: GY785 DR: 15,000 g/mol and GY785 DRS: 20,000 g/mol.

*Western blotting*

Confluent hATSC were cultured in the presence of TGF-β1 (10ng/mL) alone or in combination with GY785 DR or GY785 DRS (50μg/mL) in control medium containing low serum levels (0.5%) for 1, 4, 8 and 24 hour-periods. For each time point, cells were rapidly frozen in liquid nitrogen and conserved at -80°C until use. For western blotting, cells were thawed on ice and lysed by addition of a RIPA buffer (20mM Tris HCl, pH 7.5, 100mM potassium chloride, 1mM EDTA, 1mM EGTA, 1mM dithiothreitol, 20mM β-glycerophosphate, 2mM $Na_3VO_4$, 1mM PMSF and 1mM NaF). The protein concentration of cell lysates was determined with a Pierce Coomassie-Plus-protein assay. 30μg of total protein were resolved by sodium dodecyl sulfate-polyacrylamide gels, and proteins were transferred to a PVDF membrane following the manufacturer's protocol. Membranes were blocked and probed in 5% non-fat dry milk in PBS/Tween20. Primary antibodies were diluted 1/1000 and were detected using goat anti-rabbit (HRP)-conjugated secondary antibodies diluted 1/2000 in 5% non-fat dry milk in PBS/Tween20. The blots were visualized by Enhanced ChemiLuminescence (ECL) development using a Western blotting detection system.

As a positive control for SMAD and MAP Kinase activation, confluent osteoblastic MC3T3-E1 cells were serum starved overnight and treated for 15 minutes with anisomycin (5$\mu$g/mL), TGF-$\beta$1 (10ng/mL) or inorganic phosphate (Pi; 10mM) as previously described (Julien M et al. J Bone Miner Res. 2009;24:1856-1868).

### Statistical analysis

Each experiment was repeated at least 3 times with similar results. Results are expressed as mean $\pm$ SEM of triplicate determinations. Comparative studies of means were performed by using one-way ANOVA followed by a post hoc test (Fisher's projected least significant difference) with a statistical significance at $p<0.05$.

## Results

### Viability and proliferation of hATSC cultured in the presence of polysaccharides

To examine the viability and proliferation of hATSC cultured in the presence of 0, 25, 50, 75 and 100 $\mu$g/mL of GY785 DR or GY785 DRS, MTS activity was measured and cells were enumerated using trypan blue exclusion dye after 72 hours. As a negative control, cells were cultured in the presence of actinomycin D (5$\mu$g/mL). When treated with actinomycin D, the MTS activity and the proliferation of hATSC were significantly reduced by nearly 90%. At the concentration of 50$\mu$g/mL, GY785 DR induced a slight but significant increase in MTS activity (Figure 2A). GY785 DRS was found to trigger a dose-dependent increase in MTS activity, with a maximum at 50 $\mu$g/mL (Figure 2B). Regarding hATSC proliferation, GY785 DR did not elicit any beneficial effect (Figure 2C), whereas, the sulfated form of the molecule (GY785 DRS) at 50 and 75 $\mu$g/mL significantly increased hATSC proliferation in comparison to the control condition (Figure 2D).

These results indicate that GY785 DR and GY785 DRS have only a slight but significant effect on the viability and proliferation of hATSC. In all subsequent experiments, GY785 DR and GY785 DRS were used at the dose of 50$\mu$g/mL.

### Chondrogenic differentiation of hATSC cultured in the presence of polysaccharides

hATSC were cultured in three-dimensional pellet in the presence of control (CT) or chondrogenic (CH) medium supplemented with 50$\mu$g/mL of GY785 DR or GY785 DRS during 28 days. Chondrogenic differentiation was evaluated by a measurement of pellet volume and by the production of a GAG-containing cartilaginous matrix using alcian blue staining (Figure 3).

Surprisingly, volume estimation revealed that pellets exposed to GY785 DRS in combination with the chondrogenic medium undergo a massive increase by nearly 8-fold (Figure 3 upper panel). In addition to highlight differences in terms of GAG production between the different conditions, the gross appearance of pellets was observed after alcian blue staining. Alcian blue staining revealed that in the presence of chondrogenic medium, and regardless of polysaccharides supplementation, GAG production was up-regulated as evidenced by the intense dark blue color (Figure 3 lower panel).

To further characterize hATSC chondrogenic differentiation, cell morphology and matrix composition were then histologically examined by HES and alcian blue staining on pellets sections. In the presence of control medium (CT), HES staining allows distinguishing cell nuclei (purple) and cytoplasm (pink) but does not evidence a particular organization of the cells. On the contrary, in the presence of chondrogenic medium (CH), supplemented or not with polysaccharide, HES staining evidenced a particular structural organization of the cells within the pellet. In the external zone, cells are tangentially oriented to the surface of the pellet and in the innermost part, cells seem to be arranged radially. Moreover, in the presence of chondrogenic medium, a yellow-orange ring is visible, indicative of the synthesis of collagen fibers within the matrix. Alcian blue staining failed to reveal the presence of sulfated GAG within the matrix of pellets cultured in control medium. Interestingly, in the presence of chondrogenic medium pellet sections were strongly positive for sulfated GAG detection, especially in the presence of chondrogenic medium supplemented with GY785 DRS polysaccharide, where a denser deep blue staining can be observed.

To confirm the cartilage-like appearance of hATSC pellets evidenced by the histological observations of the present inventors, the relative expression of COL2A1, ACAN, SOX9 and COMP transcripts was determined by real-time PCR (Figure 4). The data obtained indicate that in the presence of control medium (CT) supplemented or not with polysaccharide, transcripts coding for the various chondrocyte markers could not be detected (ND) or remained at barely detectable levels. For each transcript analyzed, the expression level was significantly increased in the presence of chondrogenic medium compared to the control medium. Of interest, GY785 DRS used in combination with chondrogenic medium induced a marked increase in the expression levels of the 4 chondrogenic markers when compared to GY785 DR. GY785 DR failed to induce any additive or synergistic effect with the chondrogenic medium.

Taken together, these results show that GY785 DRS potentiates the chondrogenic differentiation of hATSC when used concomitantly with chondrogenic medium.

***Interactions between GY785 DR or GY785 DRS polysaccharides with TGF-$\beta$1/insulin of chondrogenic medium.***

To further address whether GY785 DRS may stimulate hATSC chondrogenic differentiation, several surface plasmon resonance experiments were performed. The chondrogenic medium used contained 2 major constituents: TGF-$\beta$1 (Awad HA et al. Tissue Eng. 2003;9: 1301-1312; Puetzer JL et al. Tissue Eng Part B Rev. 2010;16:435-444) and insulin (Malafaya PB et al. Tissue Eng Part A. 2010; 16:735-747; Wang CY et al. Apoptosis. 2010;15:439-449), which are known to drive the chondrogenic differentiation of MSC. To investigate whether TGF- $\beta$1 and insulin can specifically interact with GY785 DR or GY785 DRS, quantitative measurements of their potential physical interaction were performed by Biacore analysis. TGF-$\beta$ and insulin were immobilized on the chip and increasing concentrations of GY785 DR and GY785 DRS polysaccharides were injected over the chip surface. The results expressed in response units were recorded for each analyte concentration in the form of sensorgram and dissociation constant ($K_d$) was calculated. GY785 DR and GY785 DRS polysaccharides were both able to bind immobilized TGF-$\beta$. Binding affinity of GY785 DR for TGF-$\beta$ is about one-hundred-fold lower than that of GY785 DRS polysaccharide with respective $K_d$ of $3,45.10^{-8}$ and $5,5.10^{-10}$ M. No modification of the kinetic parameters of the interaction between GY785 DR or GY785 DRS in the binding of immobilized insulin was observed (data exhibiting a flat sensorgram are not shown). These results indicate that GY785 DRS can bind TGF-$\beta$1 with higher affinity than GY785 DR and none of them interacts with insulin.

***Effects of GY785 DRS on TGF-$\beta$1 signalling pathway.***

TGF-$\beta$1 is known to activate several signalling pathways including SMAD 2 (Massague J. et al. Genes Dev. 2000;14:627-644). Therefore, to address whether GY785 DRS/ TGF-$\beta$1 interaction evidenced by surface plasmon resonance may lead to specific activation of cellular events in hATSC, the potential up-regulation of TGF-$\beta$ dependent activation of SMAD 2 was determined in hATSC. To this end hATSC were exposed to 10ng/mL of TGF-$\beta$1 and 50$\mu$g/mL of GY785 DRS alone or concomitantly for 1, 4, 8 and 24 hours. MC3T3-E1 cells were used as positive controls. Cells were rapidly frozen in liquid nitrogen before lysis at 4°C. The resulting samples were analyzed by Western blot using specific antibodies against P-SMAD 2, P-ERK1/2, P-JNK1/2 and P-p38, and/or antibodies against SMAD 2 and ERK1/2. Immunoblots of cell lysates indicated that TGF-$\beta$1 alone or in association with GY785 DRS polysaccharide induced the phosphorylation of SMAD 2 in as early as 1 hour and until 24 hours. The presence of the sulfated polysaccharide alone is not sufficient to promote SMAD 2 activation and no additive or synergistic effect of GY785 DRS and TGF-$\beta$1 could be detected on the phosphorylation of SMAD 2. Since MAP kinase signalling pathways, including ERK, JNK and p38, have been largely involved in TGF-$\beta$ dependent chondrogenic differentiation (Derynck R et al. Nature. 2003;425:577-584; Arita NA et al. Biochem Biophys Res Commun. 2011), the present inventors sought to decipher whether MAPK could be activated in response to treatment with TGF-$\beta$1 and GY785 DRS.

Of particular interest, whereas ERK 1/2 phosphorylation was barely stimulated by TGF-$\beta$1 or GY785 DRS alone, the concomitant treatment of cells with TGF-$\beta$1 and GY785 DRS induced a marked up-regulation of the phosphorylation of ERK 1/2 as early as 4 hours. This stimulation was maintained up to 24 hours. Analysis of the phosphorylation of the other MAPK showed no detectable phosphorylation of either JNK or p38 in response to TGF-$\beta$1 and GY785 DRS treatment alone or combined.

To ensure the reliability of our detection method for the phosphorylation of SMAD 2, ERK1/2, JNK and p38, MC3T3-E1 cells were treated with anisomycin, Pi and TGF-$\beta$1 and used as a positive control. As expected, in these conditions, phosphorylation of p38 and JNK1/2 were observed after anisomycin treatment (Julien M et al. J Bone Miner Res. 2009;24:1856-1868), phosphorylation of ERK1/2 was observed after Pi stimulation (Julien M et al. J Bone Miner Res. 2009;24:1856-1868) and phosphorylation of SMAD 2 was observed in the presence of TGF-$\beta$1 (Lai CF et al. J Biol Chem. 2000;275:36400-36406). The phosphorylation of the various SMAD and MAPK was not associated with changes in their basal levels, suggesting that their phosphorylation resulted from the stimulation of regulatory upstream kinases.

These data suggest that TGF-$\beta$1 and GY785 DRS may act concomitantly in a synergic manner to stimulate the MAP Kinase ERK 1/2 activation in hATSC.

**Claims**

1. A liquid chondrogenic differentiation medium comprising:

   - at least one chondrogenic growth factor selected from the group consisting of transforming growth factors β, bone morphogenetic proteins, and mixtures thereof, and
   - a low-molecular-weight sulfated polysaccharide prepared from a marine native exopolysaccharide (EPS) excreted by a mesophilic marine bacterium from a deep-sea hydrothermal environment,

   wherein said low molecular-weight sulphated EPS is obtainable by a process comprising the following steps:

   (a) a step consisting of free-radical depolymerization of said native EPS so as to obtain a depolymerized EPS having a molecular weight of 5,000 to 100,000 g/mol,
   (b) a subsequent step consisting of sulfation of the depolymerized EPS, comprising adding to the depolymerized EPS at least one sulfation agent in an amount sufficient to obtain a sulfated polysaccharide having a degree of sulfate-group substitution of between 10% and 45% by weight relative to the total weight of the sulfated polysaccharide.

2. The liquid chondrogenic differentiation medium according to claim 1, wherein said low-molecular-weight sulfated polysaccharide:

   - has a molecular weight of 5,000 to 60,000 g/mol, 5,000 to 50,000 g/mol, 5,000 to 40,000 g/mol, 5,000 to 30,000 g/mol, or of 10,000 to 25,000 g/mol, and
   - has a polydispersity index of less than 5, particularly of 1.5 to 4, more particularly of less than 2.

3. The liquid chondrogenic differentiation medium according to claim 1 or 2, wherein said mesophilic marine bacterium is selected from the group consisting of bacteria of the *Alteromonas* genus, *Pseudoalteromonas* genus and *Vibrio* genus.

4. The liquid chondrogenic differentiation medium according to any one of claims 1-3, wherein said mesophilic marine bacterium is the strain GY785 of the *Alteromonas* genus.

5. The liquid chondrogenic differentiation medium according to any one of claims 1-4, wherein said medium comprises 5μg/mL to 200μg/mL, particularly 25μg/mL to 100μg/mL, more particularly 25μg/mL to 75μg/mL, most particularly 50μg/mL of said low-molecular-weight sulfated polysaccharide.

6. The liquid chondrogenic differentiation medium according to any one of claims 1-5, wherein said at least one chondrogenic growth factor is selected from the group consisting of TGF-β1, TGF-β2, TGF-β3, BMP-2, BMP-4, BMP-6, BMP-7, BMP-9, and mixtures thereof.

7. The liquid chondrogenic differentiation medium according to any one of claims 1-6, wherein said medium further comprises a fibroblast growth factor and/or an insulin-like growth factor, in particular IGF-1.

8. A kit consisting of:

   - at least one chondrogenic growth factor selected from the group consisting of transforming growth factors β, bone morphogenetic proteins, and mixtures thereof,
   - a low-molecular-weight sulfated polysaccharide prepared from a marine native exopolysaccharide (EPS) excreted by a mesophilic marine bacterium from a deep-sea hydrothermal environment, and
   - a liquid cell culture medium,

   wherein said low-molecular-weight sulfated polysaccharide is as defined in any one of claims 1-4.

**9.** A method for inducing chondrogenic differentiation in pluripotent or multipotent cells, wherein said method comprises the step of culturing pluripotent or multipotent cells with a liquid chondrogenic differentiation medium according to any one of claims 1-7.

**10.** A method for obtaining a cartilage tissue, wherein said method comprises the step of culturing pluripotent or multipotent cells with a liquid chondrogenic differentiation medium according to any one of claims 1-7.

**11.** The method according to claim 9 or claim 10, wherein said cells are human mesenchymal stem cells, in particular human adipose tissue-derived stem cells (hATSC).

**Patentansprüche**

**1.** Flüssiges Medium für chondrogene Differenzierung, umfassend:

- mindestens einen chondrogenen Wachstumsfaktor, ausgewählt aus der Gruppe bestehend aus transformierenden Wachstumsfaktoren β, knochenmorphogenetischen Proteinen, und Mischungen davon, und
- ein sulfatiertes Polysaccharid mit geringem Molekulargewicht, hergestellt aus einem marine nativen Exopolysaccharid (EPS), das durch ein mesophiles marines Bakterium aus einer hydrothermalen Tiefseeumgebung ausgeschieden wird,

wobei das sulfatierte EPS mit geringem Molekulargewicht durch ein Verfahren erhalten werden kann, das die folgenden Schritte umfasst:

a) einen Schritt, der aus freier Radikal-Depolymerisation des nativen EPS besteht, um so ein depolymerisiertes EPS mit einem Molekulargewicht von 5.000 bis 100.000 g/mol zu erhalten,
b) einen anschließenden Schritt, der aus Sulfatierung des depolymerisierten EPS besteht, umfassend Zugabe von mindestens einem Sulfatierungsmittel zu dem depolymerisierten EPS in einer ausreichenden Menge, um ein sulfatiertes Polysaccharid mit einem Sulfatgruppen-Substitutionsgrad zwischen 10 Gewichtsprozent und 45 Gewichtsprozent bezogen auf das Gesamtgewicht des sulfatierten Polysaccharids zu erhalten.

**2.** Das flüssige Medium für chondrogene Differenzierung nach Anspruch 1, wobei das sulfatierte Polysaccharid mit geringem Molekulargewicht:

- ein Molekulargewicht von 5.000 bis 60.000 g/mol, 5.000 bis 50.000 g/mol, 5.000 bis 40.000 g/mol, 5.000 bis 30.000 g/mol, oder von 10.000 bis 25.000 g/mol aufweist, und
- einen Polydispersitätsindex von weniger als 5, bevorzugt von 1.5 bis 4, stärker bevorzugt von weniger als 2 aufweist.

**3.** Das flüssige Medium für chondrogene Differenzierung nach Anspruch 1 oder 2, wobei das mesophile marine Bakterium ausgewählt ist aus der Gruppe bestehend aus Bakterien der *Alteromonas* Gattung, der *Pseudoalteromonas* Gattung und der *Vibrio* Gattung.

**4.** Das flüssige Medium für chondrogene Differenzierung nach einem der Ansprüche 1-3, wobei das mesophile marine Bakterium der Stamm GY785 der *Alteromonas* Gattung ist.

**5.** Das flüssige Medium für chondrogene Differenzierung nach einem der Ansprüche 1-4, wobei das Medium 5 μg/mL bis 200 μg/mL, bevorzugt 25 μg/mL bis 100 μg/mL, stärker bevorzugt 25 μg/mL bis 75 μg/mL, am meisten bevorzugt 50 μg/mL des sulfatierten Polysaccharids mit geringem Molekulargewicht umfasst.

**6.** Das flüssige Medium für chondrogene Differenzierung nach einem der Ansprüche 1-5, wobei der chondrogene Wachstumsfaktor ausgewählt ist aus der Gruppe bestehend aus TGF-β1, TGF-β2, TGF-β3, BMP-2, BMP-4, BMP-6, BMP-7, BMP-9, und Mischungen davon.

**7.** Das flüssige Medium für chondrogene Differenzierung nach einem der Ansprüche 1-6, wobei das Medium weiter einen Fibroblasten-Wachstumsfaktor und/oder einen Insulin-ähnlichen Wachstumsfaktor, bevorzugt IGF-1, umfasst.

**8.** Kit bestehend aus:

- mindestens einem chondrogenen Wachstumsfaktor, ausgewählt aus der Gruppe bestehend aus transformierenden Wachstumsfaktoren β, knochenmorphogenetischen Proteinen, und Mischungen davon, und
- einem sulfatierten Polysaccharid mit geringem Molekulargewicht, hergestellt aus einem marine nativen Exopolysaccharid (EPS), das durch ein mesophiles marines Bakterium aus einer hydrothermalen Tiefseeumgebung ausgeschieden wird, und
- einem flüssigen Zellkulturmedium,

wobei das sulfatierte Polysaccharid mit geringem Molekulargewicht wie in einem der Ansprüche 1-4 definiert ist.

9. Verfahren zur Induktion von chondrogener Differenzierung in pluripotenten oder multipotenten Zellen, wobei das Verfahren den Schritt Kultivieren der pluripotenten oder multipotenten Zellen mit einem flüssigen Medium für chondrogene Differenzierung nach einem der Ansprüche 1-7 umfasst.

10. Verfahren zur Gewinnung von Knorpelgewebe, wobei das Verfahren den Schritt Kultivieren der pluripotenten oder multipotenten Zellen mit einem flüssigen Medium für chondrogene Differenzierung nach einem der Ansprüche 1-7 umfasst.

11. Das Verfahren nach Anspruch 9 oder 10, wobei die Zellen humane mesenchymale Stammzellen, bevorzugt von humanem Fettgewebeabgeleitete Stammzellen (engl.: human adipose derived stem cells, hATSC) sind.

**Revendications**

1. Un milieu liquide de différenciation chondrogénique, comprenant :

- au moins un facteur de croissance chondrogénique choisi dans le groupe constitué des facteurs de croissance transformants β, des protéines de morphogénèse osseuse et de leurs mélanges, et
- un polysaccharide sulfaté de poids moléculaire bas préparé à partir d'un exopolysaccharide (EPS) natif marin excrété par une bactérie marine mésophile provenant d'un environnement hydrothermal en mer profonde,

dans lequel ledit EPS sulfaté de poids moléculaire bas peut être obtenu par un procédé comprenant les étapes suivantes :

(a) une étape de dépolymérisation radicalaire dudit EPS natif afin d'obtenir un EPS dépolymérisé possédant un poids moléculaire de 5000 à 100 000 g/mol, et
(b) une étape de sulfatation de l'EPS dépolymérisé, comprenant l'ajout à l'EPS dépolymérisé d'au moins un agent de sulfatation en une quantité suffisante pour obtenir un polysaccharide sulfaté présentant un degré de substitution de groupe sulfate situé entre 10 % et 45 % en poids par rapport au poids total du polysaccharide sulfaté.

2. Le milieu liquide de différenciation chondrogénique selon la revendication 1, dans lequel ledit polysaccharide sulfaté de poids moléculaire bas :

- présente un poids moléculaire de 5000 à 60 000 g/mol, de 5000 à 50 000 g/mol, de 5000 à 40 000 g/mol, de 5000 à 30 000 g/mol ou de 10 000 à 25 000 g/mol, et
- présente un indice de polydispersité inférieur à 5, en particulier de 1,5 à 4, plus particulièrement inférieur à 2.

3. Le milieu liquide de différenciation chondrogénique selon la revendication 1 ou la revendication 2, dans lequel ladite bactérie marine mésophile est choisie dans le groupe constitué des bactéries du genre *Alteromonas*, du genre *Pseudoalteromonas* et du genre *Vibrio.*

4. Le milieu liquide de différenciation chondrogénique selon l'une quelconque des revendications 1 à 3, dans lequel ladite bactérie marine mésophile est la souche GY785 du genre *Alteromonas*.

5. Le milieu liquide de différenciation chondrogénique selon l'une quelconque des revendications 1 à 4, dans lequel ledit milieu comprend 5 μg/ml à 200 μg/ml, en particulier 25 μg/ml à 100 μg/ml, plus particulièrement 25 μg/ml à 75 μg/ml et de manière tout à fait particulière 50 μg/ml dudit polysaccharide sulfaté de poids moléculaire bas.

**6.** Le milieu liquide de différenciation chondrogénique selon l'une quelconque des revendications 1 à 5, dans lequel ledit au moins un facteur de croissance chondrogénique est choisi dans le groupe constitué de TGF-$\beta$1, de TGF-$\beta$2, de TGF-$\beta$3, de BMP-2, de BMP-4, de BMP-6, de BMP-7, de BMP-9 et de leurs mélanges.

**7.** Le milieu liquide de différenciation chondrogénique selon l'une quelconque des revendications 1 à 6, dans lequel ledit milieu comprend en outre un facteur de croissance des fibroblastes et/ou un facteur de croissance insulino-mimétique, en particulier l'IGF-1.

**8.** Un kit comprenant :

- au moins un facteur de croissance chondrogénique choisi dans le groupe constitué des facteurs de croissance transformants $\beta$, des protéines de morphogénèse osseuse et de leurs mélanges,
- un polysaccharide sulfaté de poids moléculaire bas préparé à partir d'un exopolysaccharide (EPS) natif marin excrété par une bactérie marine mésophile provenant d'un environnement hydrothermal en mer profonde, et
- un milieu liquide de culture cellulaire,

dans lequel ledit polysaccharide sulfaté de poids moléculaire bas est tel que défini dans l'une quelconque des revendications 1 à 4.

**9.** Un procédé d'induction d'une différenciation chondrogénique chez des cellules pluripotentes ou multipotentes, dans lequel le procédé comprend l'étape de culture de cellules pluripotentes ou multipotentes avec un milieu liquide de différenciation chondrogénique selon l'une quelconque des revendications 1 à 7.

**10.** Un procédé d'obtention d'un tissu de cartilage, dans lequel ledit procédé comprend l'étape de culture de cellules pluripotentes ou multipotentes avec un milieu liquide de différenciation chondrogénique selon l'une quelconque des revendications 1 à 7.

**11.** Le procédé selon la revendication 9 ou la revendication 10, dans lequel lesdites cellules sont des cellules souches humaines du mésenchyme, en particulier des cellules souches humaines dérivées du tissu adipeux (hATSC).

**FIGURE 1**

**FIGURE 2**

21

**FIGURE 3**

**FIGURE 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009070842 A
- WO 2006003290 A
- EP 0221977 A

### Non-patent literature cited in the description

- **VINATIER et al.** *Biomaterials,* vol. 20015 (26), 6643-6651
- **REDERSTORFF et al.** *Acta Biomaterialia,* 2011, vol. 7, 2119-2130
- **REDERSTORFF et al.** *Bone,* 2011, vol. 48, 141
- *Tissue Engineering,* 2010, vol. 16 (4
- **MERCERON C. et al.** *Am J Physiol Cell Pysiol,* 2010, vol. 298 (2), 355-64
- *Nature Biotechnology,* 2007, vol. 25, 803-816
- **COLLIEC JOUAULT S. et al.** *Biochim Biophys Acta,* 2001, vol. 1528 (2-3), 141-151
- **GUEZENEC J. et al.** *Carbohydrate Polymers,* 1998, vol. 37 (1), 19-24
- **Z OU et al.** *Carbohyd. Res.,* 1998, vol. 309, 297-301
- **JENNIFER L. PUETZER.** *Tissue Engineering,* 2010, vol. 16 (4
- **VINATIER C. et al.** *Trends Biotechnol,* 2009, vol. 27 (5), 307-14
- **TAKAHASHI et al.** *Cell,* 2006, vol. 126, 663-76
- **TAKAHASHI et al.** *Cell,* 2007, 131-861, 72
- **YU et al.** *Science,* 2007, vol. 318, 1917
- **RAGUENES GH et al.** *J Appl Microbiol.,* 1997, vol. 82, 422-430
- **GUEZENNEC J et al.** *Carbohydrate Polymers.,* 1998, vol. 37, 19-24
- **COLLIEC JOUAULT S et al.** *Biochim Biophys Acta.,* 2001, vol. 1528, 141-151
- **ESTES BT et al.** *Nat Protoc.,* 2010, vol. 5, 1294-1311
- **MERCERON C et al.** *Am J Physiol Cell Physiol.,* 2010, vol. 298, 355-364
- **MERCERON C et al.** The effect of two and three dimensional cell culture on the chondrogenic potential of human adipose-derived mesenchymal stem cells after subcutaneous transplantation with an injectable hydrogel. *Cell Transplant.,* 03 February 2011
- **VINATIER C et al.** *Biomaterials,* 2005, vol. 26, 6643-6651
- **MERCERON C et al.** The effect of two and three dimensional cell Culture on the chondrogenic potential of human adipose-derived mesenchymal stem cells after subcutaneous transplantation with an injectable hydrogel. *Cell Transplant.,* 03 February 2011
- **JULIEN M et al.** *J Bone Miner Res.,* 2009, vol. 24, 1856-1868
- **AWAD HA et al.** *Tissue Eng.,* 2003, vol. 9, 1301-1312
- **PUETZER JL et al.** *Tissue Eng Part B Rev.,* 2010, vol. 16, 435-444
- **MALAFAYA PB et al.** *Tissue Eng Part A,* 2010, vol. 16, 735-747
- **WANG CY et al.** *Apoptosis,* 2010, vol. 15, 439-449
- **MASSAGUE J. et al.** *Genes Dev.,* 2000, vol. 14, 627-644
- **DERYNCK R et al.** *Nature,* 2003, vol. 425, 577-584
- **ARITA NA et al.** *Biochem Biophys Res Commun,* 2011
- **LAI CF et al.** *J Biol Chem.,* 2000, vol. 275, 36400-36406